# EUROPÄISCHE PATENTSCHRIFT

(11) **EP 3 290 063 B1**
(45) Veröffentlichungstag und Bekanntmachung des Hinweises auf die Patenterteilung: **24.10.2018**
(21) Anmeldenummer: 16187313.8
(22) Anmeldetag: 06.09.2016
(51) Int. Cl.: A61M 1/00, A61M 3/02, A61M 5/152

(54) **DRUCKREGELSYSTEM**
PRESSURE REGULATION SYSTEM
SYSTEME DE REGULATION DE PRESSION

(43) Veröffentlichungstag der Anmeldung: 07.03.2018
(73) Patentinhaber: Fritz Ruck Ophthalmologische Systeme GmbH, 52249 Eschweiler (DE)
(72) Erfinder: Klomp, Manfred, 6336 AM Hulsberg (NL)
(74) Vertreter: Schwarz & Partner Patentanwälte OG

(56) Entgegenhaltungen:
- EP-A1- 1 897 572
- US-A1- 2014 100 518
- US-A1- 2014 276 639
- US-B1- 6 491 661

## Beschreibung

Die Erfindung betrifft ein Druckregelsystem zur Abgabe einer Flüssigkeit mit einem vorgegebenen Flüssigkeitsdruck aus einer Flüssigkeitsöffnung, umfassend einen elastischen Beutel mit einer Flüssigkeitsphase und einer Gasphase, wobei die Flüssigkeitsöffnung in einer Betriebslage des elastischen Beutels im unteren Bereich des elastischen Beutels ausgebildet ist, zumindest zwei Drückelemente, welche an gegenüberliegenden Wandungen des elastischen Beutels anliegen, zumindest eine Antriebseinheit, welche zum Antreiben zumindest eines Drückelements ausgebildet ist, wobei die zumindest zwei Drückelemente durch Antreiben zumindest eines Drückelements aufeinander zu bewegbar sind, und eine Steuereinheit, welche mit der zumindest einen Antriebseinheit zum Kommunizieren verbunden ist.

Die Druckschrift US 6,491,661 B1 offenbart ein Druckregelsystem für ein Augenoperationsgerät, welches einen elastischen Beutel, zwei Drückelemente in Form von Platten, zwei Federn, ein Ventil, einen Drucksensor und zwei Antriebseinheiten umfasst. Der elastische Beutel ist über einen flexiblen Schlauch, angesteckt an eine Flüssigkeitsöffnung des elastischen Beutels, mit dem Ventil verbunden. Eine der Platten ist beweglich gelagert und die andere Platte ist mittels nicht näher ausgeführter Zusatzelemente fixiert, wobei die zwei Antriebseinheiten zum Antreiben der beweglich gelagerten Platte ausgebildet sind. Die Platten sind parallel zueinander angeordnet und liegen an gegenüberliegenden Wandungen des elastischen Beutels an, wodurch der elastische Beutel zwischen den Platten eingeklemmt ist. Durch Verkleinerung eines Abstands zwischen den Platten durch Antreiben der beweglich gelagerten Platte mittels der zwei Antriebseinheiten kann ein Druck im Beutel und infolge ein Flüssigkeitsdruck, mit dem Flüssigkeit aus dem elastischen Beutel abgegeben wird, erhöht werden, wobei mittels des Drucksensors und einer integrierten Steuerung ein vorgegebener Druck im elastischen Beutel eingehalten werden kann. Ferner weist das Druckregelsystem einen Volumenstromsensor auf, welcher eine Abgaberate an Flüssigkeit aus dem elastischen Beutel misst, wodurch eine Menge an bereits aus dem elastischen Beutel abgegebener Flüssigkeit errechenbar ist.

Bei dem aus der Druckschrift US 6,491,661 B1 bekannten Druckregelsystem hat sich als nachteilig erwiesen, dass sich vor allem bei flachen elastischen Beuteln der Flüssigkeitsdruck, mit dem die Flüssigkeit aus dem elastischen Beutel abgegeben wird, sehr schlecht regeln lässt. Schon eine kleine Verringerung des Abstands zwischen den Platten führt zu einer großen Druckänderung. Vor allem im medizinisch chirurgischen Bereich ist aber eine Feineinstellung eines von einem Druckregelsystem abgegeben Flüssigkeitsdrucks unumgänglich, um Operationsrisiken so gering wie möglich zu halten.

Es ist die Aufgabe der vorliegenden Erfindung ein Druckregelsystem bereit zu stellen, bei dem der Flüssigkeitsdruck, mit dem die Flüssigkeit aus dem elastischen Beutel abgegeben wird, besser regelbar ist.

Erfindungsgemäß wird diese Aufgabenstellung durch ein Druckregelsystem gemäß Anspruch 1 gelöst, und zwar dadurch, dass der elastische Beutel eine Gasöffnung aufweist, welche in die Gasphase reicht und dass das Druckregelsystem eine Be- und Entlüftungseinheit aufweist, welche mit der Steuereinheit zum Kommunizieren verbunden ist und welche an die Gasöffnung des elastischen Beutels anschließt, wobei die Be- und Entlüftungseinheit durch eine Gaszufuhr in den elastischen Beutel und/ oder eine Gasabfuhr aus dem elastischen Beutel einen Flüssigkeitsdruck der durch die Flüssigkeitsöffnung abgegebenen Flüssigkeit regelt.

Hierdurch ist der Vorteil erhalten, dass der Flüssigkeitsdruck, mit dem die Flüssigkeit aus dem elastischen Beutel gedrückt wird, unabhängig von einem Druck, der durch das aufeinander zu Bewegen der Platten auf die Gasphase und die Flüssigkeitsphase im elastischen Beutel ausgeübt wird, regelbar ist. Der Druck im elastischen Beutel und somit der Flüssigkeitsdruck der Flüssigkeit wird alleine nur durch die Be- und Entlüftungseinheit über eine Gaszufuhr in den elastischen Beutel und/ oder Gasabfuhr aus dem elastischen Beutel geregelt, wobei durch aufeinander zu Bewegen der Platten ein Volumen des elastischen Beutels verringert wird und infolge auch ein Volumen der Gasphase im elastischen Beutel verkleinert wird. Durch ein geringes Volumen der Gasphase passt sich der Flüssigkeitsdruck an der Flüssigkeitsöffnung sehr schnell an durch die Gaszufuhr in den elastischen Beutel und/ oder die Gasabfuhr aus den elastischen Beutel bedingte Veränderung des Drucks im elastischen Beutel an. Durch die Regelung des Drucks im Beutel über die Be- und Entlüftungseinheit ist der Flüssigkeitsdruck sehr fein einstellbar und durch das Kleinhalten des Volumens der Gasphase reagiert das Druckregelsystem sehr schnell. Vorteilhaft ermittelt die Steuereinheit über den Druck im elastischen Beutel und über einen Öffnungsquerschnitt der Flüssigkeitsöffnung die Menge an Flüssigkeit, die pro Sekunde aus dem elastischen Beutel abgegeben wird.

Bevorzugt weist der elastische Beutel eine im Wesentlichen flächige Form auf. Besonders bevorzugt ist der elastische Beutel aus einer umgefalteten Kunststofffolie gebildet, welche an ihren Rändern zusammengeschweißt ist. Hierdurch ist der Vorteil erhalten, dass die Drückelemente, die vorteilhaft auch eine flächige Form aufweisen und bevorzugt durch Platten gebildet sind, flächig an den Wandungen des elastischen Beutels anliegen. Zweckmäßig ist eine Fläche der Platten, über die die Platten an den Wandungen des elastischen Beutels anliegen, gleich groß oder größer als Flächen der Wandungen. Bevorzugt sind die Platten parallel zueinander und parallel zum elastischen Beutel angeordnet. Hierdurch ist der Vorteil erhalten, dass bei einer Bewegung der Platten aufeinander zu der gesamte elastische Beutel zusammen gedrückt wird und Ausbeulungen im elastischen Beutel großteils vermieden werden können. Ferner ist der Vorteil erhalten, dass der elastische Beutel bis auf kleine Reste komplett entleert werden kann und somit eine unnötige Verschwendung von Flüssigkeit vermieden wird.

Vorteilhaft ist die Gasöffnung durch ein erstes Rohr gebildet, welches in Betriebslage des elastischen Beutels von einer oberen Seite des elastischen Beutels in die Gasphase ragt oder welches in Betriebslage des elastischen Beutels von einer unteren Seite des elastischen Beutels in die Gasphase ragt. Die Flüssigkeitsöffnung ist vorteilhaft durch ein zweites Rohr gebildet. Durch die Ausbildung der Gasöffnung und der Flüssigkeitsöffnung als Rohre können diese bei einem elastischen Beutel, gebildet durch eine umgefaltete an den Ränder verschweißte Kunststofffolie, sehr leicht bei der Produktion zwischen die umgefaltete Kunststofffolie gelegt und mitverschweißt werden. Ragt das erste Rohr von einer unteren Seite des elastischen Beutels in die Gasphase ist der Vorteil erhalten, dass die Gasöffnung und die Flüssigkeitsöffnung an einer Seite, nämlich in Betriebslage des elastischen Beutels an der unteren Seite des elastischen Beutels, angeordnet sind, wodurch die Handhabung des elastischen Beutels erleichtert wird.

Zweckmäßig weisen die Gasöffnung und/ oder die Flüssigkeitsöffnung jeweils eine Brechsicherung auf, wobei die Gasöffnung und die Flüssigkeitsöffnung erst durch Knicken der jeweiligen Brechsicherung frei gegeben sind. Hierdurch ist der Vorteil erhalten, dass zum einen auf einen Blick die Unversehrtheit des Beutels und dessen Inhalt festgestellt werden kann und zum anderen eine Öffnung des elastischen Beutels durch Unachtsamkeit vermieden werden kann. In diesem Zusammenhang ist es auch vorteilhaft die Flüssigkeitsöffnung und/ oder die Gasöffnung mit einer Luer-Verbindung auszustatten, wodurch der elastische Beutel leicht mit anderem medizinischen Gerät oder medizinischen Elementen verbunden werden kann.

Vorteilhaft weist das Druckregelsystem Auseinanderziehelemente auf, wobei der elastische Beutel quer zu einer Bewegung des zumindest einen Drückelements beim aufeinander zu Bewegen der Drückelemente mittels den Auseinanderziehelementen auseinander ziehbar ist. Auseinanderziehelemente können zum Beispiel Federn, Aktuatoren, Seile mit Gewichten, etc. sein. Durch die Auseinanderziehelemente wird der elastische Kunststoffbeutel gestreckt, wodurch ein Zusammensacken dieses aufgrund von Schwerkraft vermieden wird und eine optimale Funktion des Druckregelsystems zu jedem Zeitpunkt gewährleistet werden kann.

In einer bevorzugten Ausführungsvariante weist das Druckregelsystem Seitenwände auf, welche jeweils seitlich an die Platten anschließen, um den elastischen Beutel zu umhüllen. Die Seitenwände können entweder starr ausgebildet sein, wobei sich beim aufeinander zu Bewegen der Drückelemente die Drückelemente an den Seitenwänden vorbei bewegen, oder die Seitenwände können auch in sich verschieblich, ähnlich einer Teleskopstange, ausgebildet sein. Durch das Vorsehen von Seitenplatten ist der Vorteil erhalten, dass bei hohen Drücken eine überproportional hohe Ausdehnung in seitlicher Richtung, insbesondere ein Ausbeulen, des elastischen Beutels verhindert wird, wodurch auch bei hohen Drücken eine gute Regelbarkeit des Flüssigkeitsdrucks gegeben ist.

In einer weiteren bevorzugten Ausführungsvariante weist das Druckregelsystem zumindest einen Sensor auf, wobei der zumindest eine Sensor zum Erkennen eines Flüssigkeitsniveaus zwischen Gasphase und Flüssigkeitsphase ausgebildet ist und an einem Drückelement angeordnet oder am elastischen Beutel appliziert ist. Hierdurch ist der Vorteil erhalten, dass durch die Steuereinheit auf Basis von Daten des Sensors die noch vorhandene Menge an Flüssigkeit im elastischen Beutel sehr genau bestimmt werden kann.

Weitere vorteilhafte Ausführungsvarianten des erfindungsgemäßen Druckregelsystems werden in weiterer Folge anhand der Figuren näher erläutert.
Figuren 1 und 2 zeigen eine erste Ausführungsvariante des erfindungsgemäßen Druckregelsystems jeweils in einer schematischen Seitenansicht.
Figur 3 zeigt die erste Ausführungsvariante des erfindungsgemäßen Druckregelsystems gemäß Figur 1 in einer schematischen Schnittansicht.
Figuren 4 und 5 zeigen jeweils eine weitere Ausführungsvariante des erfindungsgemäßen Druckregelsystems in einer schematischen Schnittansicht.
Figur 6 zeigt das erfindungsgemäße Druckregelsystem gemäß Figur 1 bei einer Verwendung in einem Augenoperationsgerät in einer schematischen Darstellung.

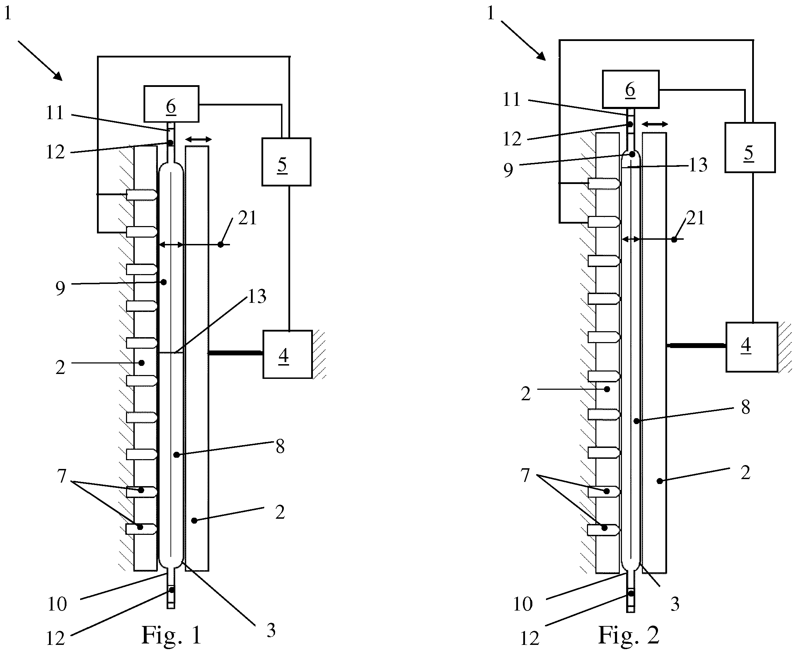

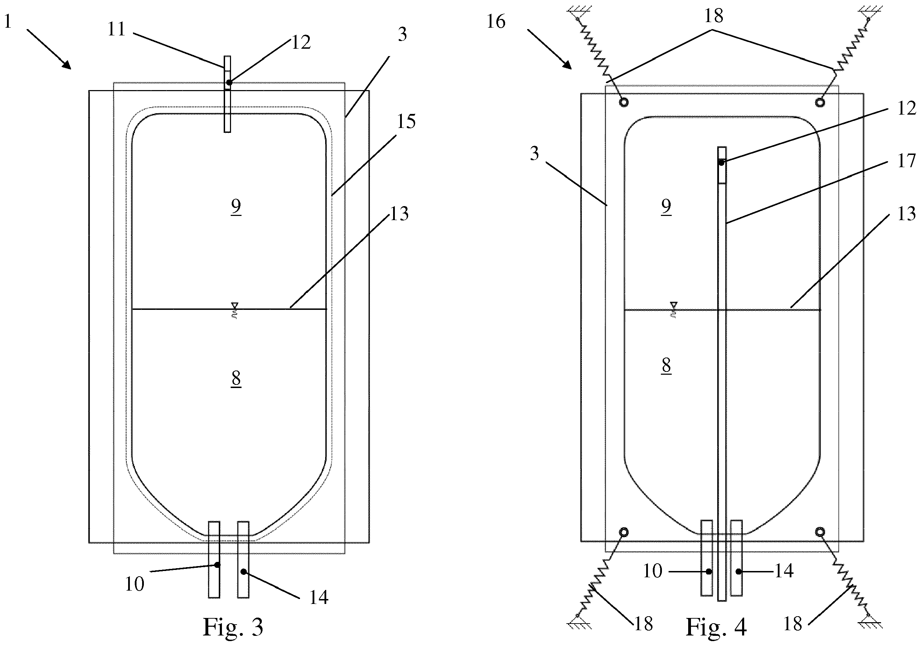

Figuren 1 und 2 zeigen eine erste Ausführungsvariante des erfindungsgemäßen Druckregelsystems 1 jeweils in einer schematischen Seitenansicht. Das Druckregelsystem 1 umfasst zwei durch Platten 2 gebildete Drückelemente, einen elastischen Beutel 3, eine Antriebseinheit 4, eine Steuereinheit 5, eine Be- und Entlüftungseinheit 6 und zehn Sensoren 7. Der elastische Beutel 3 weist eine Flüssigkeitsphase 8 und eine Gasphase 9 auf und ist in seiner Betriebslage vertikal ausgerichtet. Eine Flüssigkeit der Flüssigkeitsphase 8 kann zum Beispiel durch Infusionsflüssigkeit, insbesondere eine Kochsalzlösung, oder Irrigationsflüssigkeit gebildet sein. Ein Gas der Gasphase 9 ist vorteilhaft durch Luft gebildet. Der elastische Beutel 3 weist im unteren Bereich eine Injektionsöffnung und eine Flüssigkeitsöffnung auf, welche Flüssigkeitsöffnung durch ein zweites Rohr 10 gebildet ist und durch welche Flüssigkeit aus der Flüssigkeitsphase 8 des elastischen Beutels 3 abgegeben werden kann. Die Injektionsöffnung ist in Figur 3 dargestellt und dient zum Injizieren von Flüssigkeit oder Additiven, die zur Flüssigkeit im elastischen Beutel 3, zum Beispiel vor einem Operationsstart, zugegeben werden müssen. Die Injektionsöffnung ist durch ein drittes Rohr 14 gebildet. Im oberen Bereich des elastischen Beutels 3 ist eine Gasöffnung ausgebildet, welche Gasöffnung durch ein erstes Rohr 11 gebildet ist. Das erste Rohr 11 reicht in die Gasphase 9 des elastischen Beutels 3 und ist mit der Be- und Entlüftungseinheit 6 verbunden.

Die Platten 2 sind parallel und beabstandet zueinander angeordnet und liegen jeweils an gegenüberliegenden Wandungen des elastischen Beutels 3 an. Infolge ist der elastische Beutel 3 zwischen den Platten 2 eingeklemmt angeordnet. Eine Platte 2 ist fixiert und die andere Platte 2 ist beweglich gelagert, wobei die beweglich gelagerte Platte 2 so mittels der Antriebseinheit 4 antreibbar ist, dass die Platten 2 aufeinander zu bewegbar sind, wodurch sich ein Abstand 21 zwischen den Platten 2 verkleinern lässt. Vorteilhaft ist die Antriebseinheit 4 durch einen mit einem Elektromotor angetriebenen Zahnstangenantrieb, einen mit einem Elektromotor angetriebenen Gewindestangenantrieb, einen pneumatisch angetriebenen Zylinder oder einen hydraulisch angetriebenen Zylinder gebildet.

Sowohl die Sensoren 7, also auch die Be- und Entlüftungseinheit 6 und die Antriebseinheit 4 sind mit der Steuereinheit 5 zum Kommunizieren verbunden. Der besseren Übersichtlichkeit halber sind in Figuren 1 und 2 nicht sämtliche zehn Sensoren 7 mit der Steuereinheit 5 verbunden. Die Sensoren 7 sind durch optische Sensoren gebildet, wobei diese dann ein Sensorsignal abgeben, das die Detektion von Flüssigkeit kennzeichnet, wenn deren Messfühlerspitze im Bereich der Flüssigkeitsphase 8 an dem Beutel 3 anliegen. Derartige optische Sensoren sind dem Fachmann bekannt. Die Sensoren 7 sind in gleichen Abständen zueinander entlang einer in Betriebslage des Druckregelsystems 1 vertikalen Achse an einer Platte 2 angeordnet. Infolge kann durch die Steuereinheit 5 mittels der Sensoren 7 ein Flüssigkeitsniveau 13 im elastischen Beutel 3 detektiert werden.

Die Be- und Entlüftungseinheit 6 umfasst ein Proportionaldruckventil und eine Kompressoreinheit zum Komprimieren von Umgebungsluft.

Sowohl in dem ersten Rohr 11, als auch im zweiten Rohr 10 und im dritten Rohr 14 sind jeweils Brechsicherungen 12 ausgebildet, wobei die Rohre 10, 11 und 14 erst für den Betrieb bei einer Operation freigegeben sind, wenn die Brechsicherungen 12 geknickt wurden.

Im Folgenden wird die Funktionsweise des erfindungsgemäßen Druckregelsystems 1 näher beschrieben, wobei von einem mit der Flüssigkeit voll gefüllten elastischen Beutel 3 ausgegangen wird. Dazu wurde entweder ein neu befüllter elastischer Beutel 3 zwischen den Platten 2 angeordnet oder es wird ein bereits zwischen den Platten 2 angeordneter elastischer Beutel 3 mittels eines Zusatzgeräts über das dritte Rohr 14 mit der Flüssigkeit befüllt.

Mittels des Proportionaldruckventils der Be- und Entlüftungseinheit 6 wird gesteuert durch die Steuereinheit 5 durch eine Gaszufuhr in den elastischen Beutel 3 und/ oder eine Gasabfuhr aus dem elastischen Beutel 3 ein Druck im elastischen Beutel 3 geregelt. Der Druck im elastischen Beutel 3 wird dabei vorteilhaft direkt an der Steuereinheit 5 eingestellt. Der Flüssigkeitsdruck in dem zweiten Rohr 10 ist durch das Gewicht der Flüssigkeit geringfügig höher als der durch das Proportionaldruckventil im elastischen Beutel 3 eingestellte Druck, wobei zur Vermeidung von unerwünschtem Tropfen aus dem zweiten Rohr 10 kurzfristig mittels der Be- und Entlüftungseinheit 6 der elastische Beutel 3 mit einem Druck geringer als ein Umgebungsdruck beaufschlagt werden kann. Bei Vorsehen eines zusätzlichen Ventils, angeschlossen an das zweite Rohr 10, kann diese Funktion entfallen. Bei dem Druckregelsystem 1 wird die Abgabe von Flüssigkeit nur durch die Be- und Entlüftungseinheit 6 gesteuert, wobei schon ein geringfügig höherer Druck als der Umgebungsdruck oder ein Druck gleich dem Umgebungsdruck, je nach Menge an Flüssigkeit im elastischen Beutel 3, ausreicht, um Flüssigkeit aus dem elastischen Beutel 3 abzugeben. Die Sensoren 7 erfassen während der Abgabe von Flüssigkeit aus dem zweiten Rohr 10 kontinuierlich das Flüssigkeitsniveau 13, wobei bei Unterschreiten eines vorher vorgegebenen Flüssigkeitsniveaus 13 die Steuereinheit 5 die Antriebseinheit 4 ansteuert, die Platten 2 aufeinander zu zu bewegen, um den Abstand 21 zwischen den Platten 2 zu verringern. Durch das aufeinander zu Bewegen der Platten 2 wird ein Volumen des elastischen Beutels 3 verringert und in weiterer Folge ein Volumen der Gasphase 9 verkleinert.

Bei dem in Figur 1 dargestellten erfindungsgemäßen System 1 ist das Flüssigkeitsniveau 13 bis zur Hälfte abgesunken und der elastische Beutel 3 ist nur mehr halb gefüllt. Bei dem in Figur 2 dargestellten erfindungsgemäßen System 1 wurde der Abstand 21 zwischen den Platten 2 gegenüber dem Abstand 21 gemäß Figur 1 um die Hälfte verringert, wodurch das Flüssigkeitsniveau 13 wieder bis oben hin im elastischen Beutel 3 angestiegen ist. Sinkt das Flüssigkeitsniveau 13 in weiterer Folge wieder bis zur Hälfte ab, ist der elastische Beutel 3 nur noch mit einem Viertel der ursprünglichen Menge an Flüssigkeit befüllt. Dieser Vorgang wird solange fortgesetzt, bis der elastische Beutel 3 leer ist, oder bis der elastische Beutel 3 bis zu einer vorgegebenen Menge entleert worden ist.

Die Steuereinheit 5 ist durch Kenntnis des Abstands 21 zwischen den Platten 2 und der durch die Sensoren 7 gemessenen Lage des Flüssigkeitsniveaus 13 dazu ausgebildet die Menge an im elastischen Beutel 3 vorhandenen Flüssigkeit zu erfassen, wobei eine Messgenauigkeit der Menge an vorhandener Flüssigkeit bei Verringerung des Abstands 21 zunimmt. Durch die Bewegung der Platten 2 aufeinander zu und die daraus resultierende Verringerung des Abstands 21 zwischen den Platten 2 folgt somit nicht nur eine Verringerung des Volumens der Gasphase 9, wodurch eine Trägheit des Systems 1 bei Druckveränderungen vermieden wird, sondern es wird auch die Messgenauigkeit der Menge an Flüssigkeit, die im elastischen Beutel 3 noch vorhanden ist, bei gleichbleibender Anzahl an Sensoren 7 erhöht.

In einer weiteren Ausführungsvariante sind die Sensoren 7 durch kapazitive Sensoren gebildet, wobei jeweils ein kapazitiver Sensor zwei Sensorelemente aufweist, die gegenüberliegend jeweils an den Platten 2 befestigt sind.

Figur 3 zeigt die erste Ausführungsvariante des erfindungsgemäßen Druckregelsystems 1 nach Figur 1 in einer schematischen Schnittansicht. Der elastische Beutel 3 besteht aus einer einstückigen Kunststofffolie, die umgefaltet ist und an Rändern 15 der Kunststofffolie verschweißt ist.

Figur 4 zeigt eine weitere Ausführungsvariante des erfindungsgemäßen Druckregelsystems 16 in einer schematischen Frontansicht. Das Druckregelsystem 16 unterscheidet sich zu dem in den Figuren 1 bis 3 gezeigten Druckregelsystem 1 dadurch, dass es Auseinanderziehelemente in Form von Federn 18 aufweist. Mittels der Federn 18 wird der elastische Beutel 3 quer zu der Bewegung der beweglich gelagerten Platten 2 beim aufeinander zu Bewegen der Platten 2 auseinander gezogen, wodurch ein Zusammenfallen bzw. ein Zusammenfalten des elastischen Beutels 3 verhindert wird und dieser im Wesentlichen seine Form behält.

Ferner reicht bei dem Druckregelsystem 16 ein die Gasöffnung bildendes erstes Rohr 17 von einer unteren Seite des elastischen Beutels 3 in die Gasphase 9. Hierdurch ist der Vorteil erhalten, dass sämtliche Anschlüsse auf einer Seite des elastischen Beutels 3 angeordnet sind.

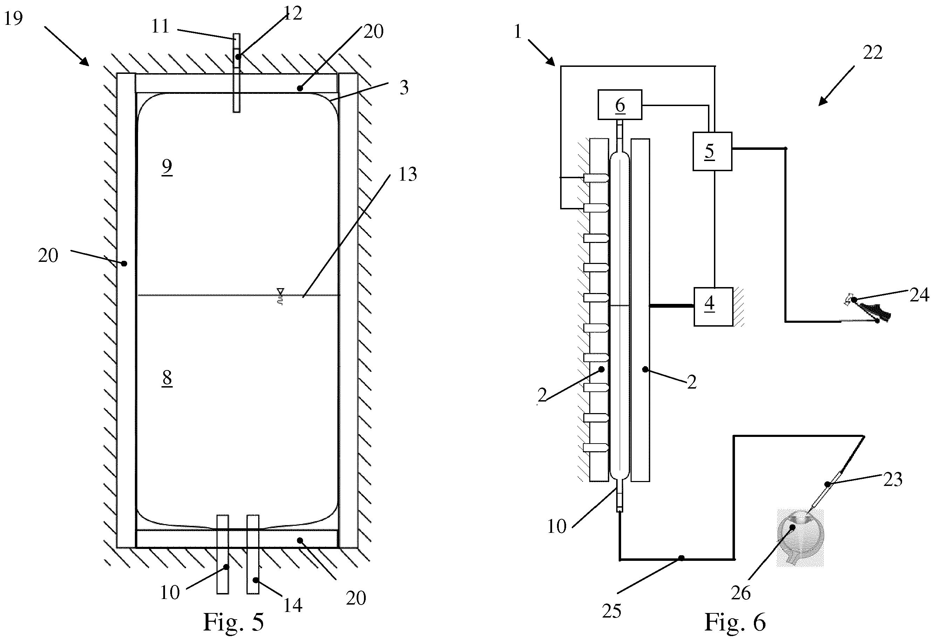

Figur 5 zeigt eine weitere Ausführungsvariante des erfindungsgemäßen Druckregelsystems 19 in einer schematischen Frontansicht. Das Druckregelsystem 19 unterscheidet sich zu dem in Figur 1 gezeigten Druckregelsystem 1 dadurch, dass das Druckregelsystem 19 Seitenwände 20 aufweist. Die Seitenwände 20 sind jeweils an Zusatzelementen, insbesondere einem Rahmen, fixiert, und sind durch weitere Platten gebildet, wobei die angetriebene Platte 2 sich relativ zu den weiteren Platten bewegen kann. Hierdurch ist der Vorteil erhalten, dass ein seitliches Ausweichen des elastischen Beutels 3 oder ein Ausbeulen des elastischen Beutels 3 auch bei hohen Drücken vermieden wird, wodurch der Flüssigkeitsdruck auch bei hohen Drücken sehr gut regelbar ist und es zu keiner Trägheit des Systems 19 aufgrund der Ausdehnung des elastischen Beutels 3 kommen kann.

Figur 6 zeigt das erfindungsgemäße Druckregelsystem 1 gemäß Figur 1 bei einer Verwendung in einem Augenoperationsgerät 22 in einer schematischen Darstellung. Das Augeoperationsgerät 22 umfasst zusätzlich zum Druckregelsystem 1 ein chirurgisches Handstück 23 und eine Regeleinrichtung 24, wobei das chirurgische Handstück 23 mittels eines Schlauchs 25 direkt an das zweite Rohr 10 angeschlossen ist und ein nicht dargestelltes Regelventil aufweist. Das Regelventil kann zum Beispiel durch ein Magnetventil gebildet sein. Die Regeleinheit 24 ist durch ein Fußpedal gebildet, welches zum Kommunizieren mit der Steuereinheit 5 verbunden ist. Mittels der Regeleinheit 24 kann der Druck im elastischen Beutel 3 und daraus resultierend ein Irrigationsdruck verändert werden, mittels welchem die Flüssigkeit im elastischen Beutel 3 über das chirurgischen Handstück 23 an ein Auge 26 abgegeben wird.

Vorteilhaft wird während des gesamten operativen Eingriffs am Auge 26, insbesondere auch bei einer Veränderung des Abstands 21 zwischen den Platten 2, der Druck im elastischen Beutel 3 durch die Be- und Entlüftungseinheit 6 konstant gehalten.

In einer weiteren Ausführungsvariante erfolgt eine Regelung des Drucks im elastischen Beutel 3 nur während die Platten 2 still stehen. Während einer Änderung des Abstands 21 zwischen den Platten 2 wird sowohl die Regelung des Drucks, als auch die Entnahme von Flüssigkeit aus dem elastischen Beutel 3 unterbrochen. Bevorzugt wird bei dieser Ausführungsvariante eine Veränderung des Abstands 21 der Platten 2 zur Minimierung des Gasvolumens durchgeführt, wenn ein das Augenoperationsgerät 22 nutzender Operateur absetzt und dadurch kurzzeitig den chirurgischen Eingriff unterbricht.

In einer weiteren Ausführungsvariante ist die Be- und Entlüftungseinheit 6 durch ein durch die Steuereinheit 5 ansteuerbares Ventil gebildet.

## Patentansprüche

1. Druckregelsystem (1, 16, 19) zur Abgabe einer Flüssigkeit mit einem vorgegebenen Flüssigkeitsdruck aus einer Flüssigkeitsöffnung (10), umfassend
einen elastischen Beutel (3) mit einer Flüssigkeitsphase (8) und einer Gasphase (9), wobei die Flüssigkeitsöffnung (10) in einer Betriebslage des elastischen Beutels (3) im unteren Bereich des elastischen Beutels (3) ausgebildet ist,
zumindest zwei Drückelemente (2), welche an gegenüberliegenden Wandungen des elastischen Beutels (3) anliegen,
zumindest eine Antriebseinheit (4), welche zum Antreiben zumindest eines Drückelements ausgebildet ist, wobei die zumindest zwei Drückelemente durch Antreiben zumindest eines Drückelements aufeinander zu bewegbar sind, und
eine Steuereinheit (5), welche mit der zumindest einen Antriebseinheit (4) zum Kommunizieren verbunden ist,
**dadurch gekennzeichnet, dass** der elastische Beutel (3) eine Gasöffnung aufweist, welche in die Gasphase (9) reicht und dass das Druckregelsystem (1, 16, 19) eine Be- und Entlüftungseinheit (6) aufweist, welche mit der Steuereinheit (5) zum Kommunizieren verbunden ist und welche an die Gasöffnung des elastischen Beutels (3) anschließt, wobei die Be- und Entlüftungseinheit (6) durch eine Gaszufuhr in den elastischen Beutel (3) und/ oder eine Gasabfuhr aus dem elastischen Beutel (3) den Flüssigkeitsdruck der durch die Flüssigkeitsöffnung (10) abgegebenen Flüssigkeit regelt.

2. Druckregelsystem (1, 16, 19) nach Anspruch 1, **dadurch gekennzeichnet, dass** ein Druck im elastischen Beutel (3) bei sich aufeinander zu bewegenden Drückelementen (2) durch die Be- und Entlüftungseinheit (6) konstant gehalten ist.

3. Druckregelsystem (1, 16, 19) nach Anspruch 1 oder 2, **dadurch gekennzeichnet, dass** der elastische Beutel (3) eine im Wesentlichen flächige Form aufweist.

4. Druckregelsystem (1, 16, 19) nach einem der Ansprüche 1 bis 3, **dadurch gekennzeichnet, dass** der elastische Beutel (3) aus einer umgefalteten Kunststofffolie gebildet ist, welche an ihren Rändern (15) zusammengeschweißt ist.

5. Druckregelsystem (1, 16, 19) nach einem der Ansprüche 1 bis 4, **dadurch gekennzeichnet, dass** die Gasöffnung durch ein erstes Rohr (12, 17) gebildet ist, welches in Betriebslage des elastischen Beutels (3) von einer oberen Seite des elastischen Beutels (3) in die Gasphase (9) ragt oder welches in Betriebslage des elastischen Beutels (3) von einer unteren Seite des elastischen Beutels (3) in die Gasphase (9) ragt.

6. Druckregelsystem (1, 16, 19) nach einem der Ansprüche 1 bis 5, **dadurch gekennzeichnet, dass** die Gasöffnung und/ oder die Flüssigkeitsöffnung (10) jeweils eine Brechsicherung (12) aufweisen, wobei die Gasöffnung und die Flüssigkeitsöffnung (10) erst durch Knicken der jeweiligen Brechsicherung (12) frei gegeben sind.

7. Druckregelsystem (1, 16, 19) nach einem der Ansprüche 1 bis 6, **dadurch gekennzeichnet, dass** die Gasöffnung und/ oder die Flüssigkeitsöffnung (10) eine Luer-Verbindung aufweist/en.

8. Druckregelsystem (1, 16, 19) nach einem der Ansprüche 1 bis 7, **dadurch gekennzeichnet, dass** das Druckregelsystem (1, 16, 19) Auseinanderziehelemente (18) aufweist, wobei die Auseinanderziehelemente (18) den elastischen Beutel (3) quer zu einer Bewegung des zumindest einen Drückelements beim aufeinander zu Bewegen der Drückelemente auseinander ziehen.

9. Druckregelsystem (1, 16, 19) nach einem der Ansprüche 1 bis 8, **dadurch gekennzeichnet, dass** die zumindest zwei Drückelemente durch Platten (2) gebildet sind, die parallel zueinander angeordnet sind.

10. Druckregelsystem (1, 16, 19) nach Anspruch 9, **dadurch gekennzeichnet, dass** das Druckregelsystem (1, 16, 19) Seitenwände (20) aufweist, welche jeweils seitlich an die Platten (2) anschließen, um den elastischen Beutel (3) zu umhüllen.

11. Druckregelsystem (1, 16, 19) nach einem der Ansprüche 1 bis 10, **dadurch gekennzeichnet, dass** das Druckregelsystem (1, 16, 19) zumindest einen Sensor (7) aufweist, wobei der zumindest eine Sensor (7) zum Erkennen eines Flüssigkeitsniveaus (13) zwischen Gasphase (9) und Flüssigkeitsphase (8) ausgebildet ist und an einem Drückelement angeordnet oder am elastischen Beutel (3) appliziert ist.

## Claims

1. A pressure regulation system (1, 16, 19) for discharging a liquid having a predetermined liquid pressure from a liquid opening (10), comprising
an elastic bag (3) having a liquid phase (8) and a gas phase (9), wherein the liquid opening (10) in an operational position of the elastic bag (3) is formed in the lower area of the elastic bag (3),
at least two push elements (2), which abut at opposite walls of the elastic bag (3),
at least one drive unit (4), which is configured to drive at least one push element, wherein the at least two push elements may be moved towards one another by driving at least one push element, and
a control unit (5), which is connected to the at least one drive unit (4) for communication, **characterized in that** the elastic bag (3) has a gas opening, which extends into the gas phase (9), and that the pressure regulation system (1, 16, 19) has a ventilation unit (6), which is connected to the control unit (5) for communication and which connects to the gas opening of the elastic bag (3), wherein the ventilation unit (6) regulates the liquid pressure of the liquid discharged through the liquid opening (10) by means of a gas supply into the elastic bag (3) and/or a gas discharge from the elastic bag (3).

2. A pressure regulation system (1, 16, 19) according to claim 1, **characterized in that** a pressure in the elastic bag (3) is kept constant by the ventilation unit (6) in the case of push elements (2) moving towards one another.

3. A pressure regulation system (1, 16, 19) according to claim 1 or 2, **characterized in that** the elastic bag (3) has an essentially plane shape.

4. A pressure regulation system (1, 16, 19) according to any of claims 1 to 3, **characterized in that** the elastic bag (3) is formed from a plastic film that is folded over, which is sealed at the edges (15) thereof.

5. A pressure regulation system (1, 16, 19) according to any of claims 1 to 4, **characterized in that** the gas opening is formed by a first tube (12, 17), which in the operational position of the elastic bag (3) projects from an upper side of the elastic bag (3) into the gas phase (9) or which in the operational position of the elastic bag (3) projects from a lower side of the elastic bag (3) into the gas phase (9).

6. A pressure regulation system (1, 16, 19) according to any of claims 1 to 5, **characterized in that** the gas opening and/or the liquid opening (10) each have a protection against break (12), wherein the gas opening and the liquid opening (10) are released only upon cracking of the respective protection against break (12).

7. A pressure regulation system (1, 16, 19) according to any of claims 1 to 6, **characterized in that** the gas opening and/or the liquid opening (10) have a Luer connection.

8. A pressure regulation system (1, 16, 19) according to any of claims 1 to 7, **characterized in that** the pressure regulation system (1, 16, 19) has extension elements (18), wherein the extension elements (18) extend the elastic bag (3) transversely to a movement of the at least one push element when the push elements move towards one another.

9. A pressure regulation system (1, 16, 19) according to any of claims 1 to 8, **characterized in that** the at least two push elements are formed by plates (2), which are arranged in parallel to one another.

10. A pressure regulation system (1, 16, 19) according to claim 9, **characterized in that** the pressure regulation system (1, 16, 19) has side walls (20), which each laterally abut the plates (2) in order to enclose the elastic bag (3).

11. A pressure regulation system (1, 16, 19) according to any of claims 1 to 10, **characterized in that** the pressure regulation system (1, 16, 19) has at least one sensor (7), wherein the at least one sensor (7) is configured to detect a liquid level (13) between gas phase (9) and liquid phase (8) and is arranged on a push element or applied onto the elastic bag (3).

## Revendications

1. Système de régulation de pression (1, 16, 19) pour distribuer un liquide à une pression donnée du liquide hors d'une ouverture pour liquide (10), comportant
un sachet élastique (3) contenant une phase liquide (8) et une phase gazeuse (9), l'ouverture pour liquide (10) étant ménagée, en configuration de fonctionnement du sachet élastique (3), dans la zone inférieure du sachet élastique (3),
au moins deux éléments presseurs qui s'appuient contre des parois opposées du sachet élastique (3),
au moins une unité d'entraînement (4) qui est réalisée pour entraîner au moins un élément presseur, lesdits au moins deux éléments presseurs étant mobiles l'un vers l'autre par entraînement d'au moins un élément presseur,
une unité de commande (5) qui est reliée à ladite au moins une unité d'entraînement (4) pour communiquer avec celle-ci,
**caractérisé en ce que**
le sachet élastique (3) présente une ouverture pour gaz qui va jusque dans la phase gazeuse (9), et **en ce que** le système de régulation de pression (1, 16, 19) comprend une unité d'aération et de ventilation (6) qui est reliée à l'unité de commande (5) pour communiquer avec celle-ci et qui se raccorde à l'ouverture pour gaz du sachet élastique (3), l'unité d'aération et de ventilation (6) régulant la pression de liquide du liquide distribué par l'ouverture pour liquide (10) au moyen d'une alimentation en gaz dans le sachet élastique (3) et/ou une d'évacuation de gaz hors du sachet élastique (3).

2. Système de régulation de pression (1, 16, 19) selon la revendication 1, **caractérisé en ce qu'**une pression dans le sachet élastique (3) est maintenue constante par l'unité d'aération et de ventilation (6) lorsque les éléments presseurs (2) se déplacent l'un vers l'autre.

3. Système de régulation de pression (1, 16, 19) selon la revendication 1 ou 2, **caractérisé en ce que** le sachet élastique (3) présente une forme sensiblement plane.

4. Système de régulation de pression (1, 16, 19) selon l'une des revendications 1 à 3, **caractérisé en ce que** le sachet élastique (3) est formé par une feuille pliée en matière plastique qui est soudée au niveau de ses bords (15).

5. Système de régulation de pression (1, 16, 19) selon l'une des revendications 1 à 4, **caractérisé en ce que** l'ouverture pour gaz est formée par un premier tube (12, 17) qui, en configuration de fonctionnement du sachet élastique (3), pénètre dans la phase gazeuse (9) depuis un côté supérieur du sachet élastique (3) ou qui, en configuration de fonctionnement du sachet élastique (3), pénètre dans la phase gazeuse (9) depuis un côté inférieur du sachet élastique (3).

6. Système de régulation de pression (1, 16, 19) selon l'une des revendications 1 à 5, **caractérisé en ce que** l'ouverture pour gaz et/ou l'ouverture pour liquide (10) présentent chacune un moyen de sécurité (12) à la rupture, l'ouverture pour gaz et l'ouverture pour liquide (10) n'étant libérées que par gauchissement du moyen de sécurité à la rupture respectif (12).

7. Système de régulation de pression (1, 16, 19) selon l'une des revendications 1 à 6, **caractérisé en ce que** l'ouverture pour gaz et/ou l'ouverture pour liquide (10) présente(nt) un raccord Luer.

8. Système de régulation de pression (1, 16, 19) selon l'une des revendications 1 à 7, **caractérisé en ce que** le système de régulation de pression (1, 16, 19) présente des éléments d'étirage (18), les éléments d'étirage (18) étirant le sachet élastique (3) transversalement à un mouvement dudit au moins un élément presseur lorsque les éléments presseurs se déplacent l'un vers l'autre.

9. Système de régulation de pression (1, 16, 19) selon l'une des revendications 1 à 8, **caractérisé en ce que** lesdits au moins deux éléments presseurs sont formés par des plaques (2) qui sont agencées parallèlement l'une à l'autre.

10. Système de régulation de pression (1, 16, 19) selon la revendication 9, **caractérisé en ce que** le système de régulation de pression (1, 16, 19) présente des parois latérales (20) qui se raccordent chacune latéralement aux plaques (2) pour envelopper le sachet élastique (3).

11. Système de régulation de pression (1, 16, 19) selon l'une des revendications 1 à 10, **caractérisé en ce que** le système de régulation de pression (1, 16, 19) présente au moins un capteur (7), ledit au moins un capteur (7) étant réalisé pour reconnaître un niveau de liquide (13) entre la phase gazeuse (9) et la phase liquide (8) et étant agencé sur un élément presseur ou étant appliqué contre le sachet élastique (3).
